# EUROPEAN PATENT SPECIFICATION

(11) **EP 2 386 252 B1**
(45) Date of publication and mention of the grant of the patent: **07.04.2021**
(21) Application number: 10162574.7
(22) Date of filing: 11.05.2010
(51) Int. Cl.: A61B 17/06

(54) **Use of a continuous-filament thread having a plurality of barbs for the production of sutures**
Verwendung einer Endlosfaser mit mehreren Widerhaken zur Herstellung von Nahtfäden
Utilisation d'un fil à filament continu doté de plusieurs crans pour la production de fils

(43) Date of publication of application: 16.11.2011
(73) Proprietor: Aesculap AG, 78532 Tuttlingen/Donau (DE)
(72) Inventor: Volker, Friedrich, 08191 Barcelona/Rubi (ES); Odermatt, Erich Dr., 8200 Schaffhausen (CH); Funk, Lutz Dr., 08172 Sant Cugat der Vallés (ES); Casanovas Albalate, Marta, 08950 Esplugues de Llobregat Barcelona (ES)
(74) Representative: Patentanwälte Ruff, Wilhelm, Beier, Dauster & Partner mbB

(56) References cited:
- EP-A1- 2 020 209
- WO-A1-2006/037399
- WO-A1-2010/052006
- WO-A1-2010/127873
- WO-A1-2010/127874
- WO-A2-03/017850
- WO-A2-2009/086172
- WO-A2-2009/097556
- WO-A2-2009/105663
- WO-A2-2009/132284
- DE-A1-102007 058 256
- FR-A1- 2 914 178
- US-A1- 2003 149 447
- US-A1- 2007 257 395
- US-A1- 2008 281 357
- US-A1- 2009 099 597

## Description

The present invention relates to the use of a continuous-filament thread having a plurality of barbs.

Various surgical methods employing sutures have been used in the past for closing or binding together wounds in human or animal tissue. More specifically, the surgeon may use a surgical needle with an attached conventional suture to pierce the tissue alternately on opposing faces of the wound and thus sew the wound closed. Usually, the sutures are knotted in order to obtain a secure fixing in the tissue. Care has to be taken to ensure that the wounds to be closed are sutured with an optimal force at the wound margins. If the wound margins are sutured too loosely and too irregularly, for example, there is in principle a risk of increased scaring or dehiscence. By contrast, if the wound margins are sewed too strongly, there is a danger of the circulation of blood in the wound margins being restricted, which can result in necrotic changes in the surrounding tissue area.

In addition to the risk of possible complications, in particular further surgical interventions, there is therefore always a degree of risk of the wound repair, based on knotting of sutures, leading to impaired healing and to unsatisfactory cosmetic results in the patients concerned. Another consideration is that several knots often have to overlap in order to achieve a secure knot hold. This entails introducing a large amount of material into the area of the wound, which may elicit tissue erosion and foreign-body reactions.

Sutures which, in contrast to conventional sutures, do not have to be knotted have long been known under the term "barbed sutures". Since the time of their conception, barbed sutures have offered numerous advantages over closing wounds with conventional sutures. A barbed suture typically includes an elongated body that has spaced barbs which project from the body surface along the body length. The barbs are arranged on the elongate body in such a way that the suture can be pulled through the tissue along the direction of the barbs without any great resistance and without tissue trauma. When a pull is exerted in the opposite direction, however, the barbs stand upright and anchor themselves, and therefore also the suture, in the surrounding tissue area. This ensures that the suture cannot be pulled back through the incision channel. Thus, the main advantage of barbed sutures has been the provision of a non-slip attribute. Barbed sutures are described, for example, in the documents US 3,123,077 A, EP 1 559 266 B1, EP 1 560 683 B1 and EP 1 556 946 B1.

With respect to barbed sutures, the barbs are often arranged in a symmetrical fashion on the suture. For instance, the barbs may be formed in a helical pattern on the sutures, wherein the helix has a defined twist direction, cut depth and cut distance. However, such a helical disposition of the barbs may lead to a coil of the suture depending on the length of the incision, which may have an adverse effect on the scaring.

A further disadvantage that is associated with barbed sutures relates to their producing process. Normally, barbed sutures having distinct properties, in particular in view of barb geometry, have to be produced in an elaborate and time-consuming process.

Continuous filament threads for obtaining barbed sutures are known from WO 2009/097556 A2, WO 2009/105663 A2 and WO 03/017850 A2. The object of the present invention is therefore to make available a use of a barbed material which avoids disadvantages known from the prior art. The barbed material according to the present invention should allow for a comfortable provision of barbed sutures having distinct properties depending on the wound to be closed by the barbed suture.

Accordingly, the present invention provides a use of a continuous-filament thread according to claim 1.

The term "continuous-filament thread" and "continuous-filament thread body", respectively as used according to the invention, preferably relates to a thread and thread body, respectively that is unprocessed at least in view of its length ("endless-filament thread" and "endless-filament thread body", respectively).

The term "barbs" as used according to the invention encompasses at least one barb.

The term "plurality of barbs" as used according to the invention encompasses at least two or more barbs.
The term "thread" and "thread body", respectively as used according to the invention, may include a fibre, a yarn, a strand, a monofilament, in particular a pseudo-monofilament, or a multifilament, in particular a braided or intertwined multifilament.

The continuous-filament thread body comprises an alternating or a random barb configuration. The term "configuration" according to the invention is to be understood in its broadest sense and encompasses the design of the barbs. More specifically, the barbs may have an alternating or random configuration in view of barb length, barb height, cutting depth, cutting distance, cutting angle, cutting angle offset and, combinations thereof. In view of producing barbed sutures having alternating or random barb configurations, the continuous-filament thread body comprises an alternating or random barb configuration along its length. Thus, a multiplicity of barbed sutures having distinct barb configurations may be produced from a single continuous-filament thread according to the present invention.

The continuous-filament thread body comprises a random barb configuration.

In a further preferred embodiment, the barbs have a shape that is selected from the group consisting of escutcheon-shape, shield-shape, scale-shape, wedge-shape, thorn-shape, W-shape, arrow-shape, spike-shape, tin-shape, V-shape and, combinations thereof. Further, the barbs are preferably pointed or tapered at their free ends. Furthermore, the barbs may have a multi-tip configuration, in particular a twin-tip configuration. An example for barbs having a twin-tip configuration is the above mentioned W-shaped formation of barbs. Barbs having a twin-tip configuration may in particular be based on flat cuts into the continuous-filament thread body, preferably formed with a small angular offset and in small intervals from each other.

In view of the production of different barbed sutures from a single continuous-filament thread according to the invention, the barbs are arranged in alternating or random dispositions on the continuous-filament thread body.

More specifically, the barbs have a disposition on the continuous-filament thread body that is selected from the group consisting of a row disposition, a staggered disposition, an overlapping disposition, an offset disposition, an offset and partially overlapping disposition, a zigzag disposition, a random or arbitrary disposition, a meander-like disposition, a serpentine-like disposition, a sinus-like disposition, a spiral disposition, a helical disposition, and combinations thereof.

According to a further embodiment, the barbs are arranged in the form of left-handed and/or right-handed spirals and/or helices on the continuous-filament thread body. More specifically, left-handed and/or right-handed spirals and/or helices may be present on the continuous-filament thread body in a different or varying, in particular alternating or random, disposition. For example, a spiral or helical barb disposition having a different or varying, in particular alternating or random, spiral or helical twist, contributes to the reduction of suture coiling. Thus, adverse interactions between a tissue and a coiled suture may be significantly reduced, thereby enhancing wound healing.

In a preferred embodiment, the barbs are arranged in a unidirectional disposition on the continuous-filament thread body. The term "unidirectional disposition" as used herein, relates to a barb disposition, wherein the barbs are orientated in the same direction on the continuous-filament thread body.

More preferably, the barbs are arranged in a multidirectional, in particular bidirectional, disposition on the continuous-filament thread body. According to the invention, the term "multidirectional disposition" relates to a disposition of barbs, in which the barbs are oriented or aligned in multiple directions on the continuous-filament thread body. Accordingly, the term "bidirectional disposition" relates to a disposition of barbs, in which the barbs are oriented or aligned in two different directions. Preferably, the barbs are orientated or aligned in opposing directions. More preferably, seen in the longitudinal direction of the continuous-filament thread body, the barbs for a first continuous-filament thread body section are preferably formed in the direction of a second continuous-filament thread body section and, for the second continuous-filament thread body section, are formed in the direction of the first continuous-filament thread body section.

The continuous-filament thread body may comprise in a further embodiment repeating units of unidirectionally arranged barbs.

As an alternative or in combination, the continuous-filament thread body may comprise repeating units of multidirectionally arranged barbs. Preferably, the continuous-filament thread body comprises repeating units of bidirectionally arranged barbs. In particular, each unit may comprise a first section and a second section, wherein, seen in the longitudinal direction of the continuous-filament thread body, the barbs of the first section are orientated in the direction of the second section and the barbs of the second section are orientated in the direction of the first section.

Generally, the continuous-filament thread body may comprise at least two, in particular two or three, multidirectional dispositions of barbs.

In a further embodiment, the continuous-filament thread body may comprise at least two, in particular two, bidirectional dispositions of barbs on its surface. It is particularly preferable if, in relation to a first bidirectional disposition of barbs, a second bidirectional disposition of barbs is formed on the continuous-filament thread body at approximately 180° in the circumferential direction and preferably offset in relation to the first bidirectional disposition.

It is also conceivable that the continuous-filament thread body comprises three bidirectional dispositions of barbs. In this case, it is preferable if, in relation to a first bidirectional disposition of barbs, a second bidirectional disposition of barbs is formed on the continuous-filament thread body at approximately 120° in the circumferential direction and preferably offset in relation to the first bidirectional disposition, which second bidirectional disposition of barbs is in turn formed at approximately 120° in the circumferential direction and preferably offset in relation to a third bidirectional disposition of barbs, such that the third bidirectional disposition of barbs is likewise formed at approximately 120° in the circumferential direction and preferably offset in relation to the first bidirectional disposition of barbs.

It is further within the scope of the present invention that the continuous-filament thread body may comprise more than three, in particular four, five or six, multidirectional, in particular bidirectional, dispositions of barbs on the continuous-filament thread body.

According to an especially preferred embodiment, the continuous-filament thread body comprises sections that are free of barbs, in the following also denoted as barbless sections. The barbless sections may generally serve as potentially cutting areas so as to obtain barbed sutures from the continuous-filament thread body of the present invention. Preferably, the barbless sections at least are also present in a barbed suture that is obtained from the continuous-filament thread. More specifically, the barbless sections may serve as junction areas for joining lengths or sections, in particular sutures bodies, obtained by cutting the continuous-filament thread body. Furthermore the barbless sections may serve as attachment areas for attaching medical instruments, in particular inserting instruments such as a surgical needle, and/or for attaching medical devices such as meshes to a barbed suture that may be obtained from the continuous filament thread. Furthermore, the barbless sections may advantageously facilitate the identification of the centre of a barbed suture that may be obtained from the continuous-filament thread.

In a further embodiment, terminal ends of the continuous-filament thread body may be free of barbs.

More specifically, the continuous-filament thread body may comprise a different or varying, in particular an alternating or a random, disposition of barbed sections and barbless sections.

It is especially preferred that the continuous-filament thread body comprises sections having barbs that are interspaced by sections having no barbs. In other words, it is especially preferred that the continuous-filament thread body may comprise an alternating disposition of sections having barbs (barbed sections) and sections having no barbs (barbless sections). The barbed sections may comprise unidirectionally or multidirectionally, in particular bidirectionally, arranged barbs.

The continuous-filament thread body is designed as a hollow continuous-filament thread body, in particular as a tubular continuous-filament thread body, preferably as continuous-tube or continuous-hose. The term "continuous-tube" and "continuous-hose", respectively preferably relates to a tube and hose, respectively that is unprocessed at least in view of its length. Preferably, the hollow continuous-filament thread body comprises a closed wall, wherein the continuous-filament thread body preferably comprises open ends. A hollow, in particular tubular, continuous-filament thread body may be produced, by way of example, by means of an extrusion process. The barbs may project into the interior and/or exterior of the hollow continuous-filament thread body. Particularly, the barbs may be designed as cuts into the hollow continuous-filament thread body, wherein the barbs preferably do not break through the wall of the hollow continuous-filament thread body. As an alternative or in combination, the barbs may be designed as breakthroughs, i.e. the barbs are formed completely breaking through the wall of the hollow continuous-filament thread body.

Further, the continuous-filament thread body may not be limited in view of its cross section. In general, a circular cross section of the continuous-filament thread body is preferred. However, other cross-sectional shapes are likewise conceivable. For example, the continuous-filament thread body can have an oval, triangular, rosette-like, square, trapezoidal, rhomboid, pentagonal, hexagonal, star-shaped or cross-shaped cross section. Such cross-sectional shapes can readily be formed by means of suitable extrusions dies, which can be produced specific to a customer with any desired cross-sectional shape.

It may be within the scope of the present invention that the barbs are configured in at least two, in particular two, three, four, five or more, sets, each set having a barb configuration different from the barb configuration of the other set.

Preferably, the continuous-filament thread body is made from a material selected from the group consisting of a bio-absorbable material, a non-absorbable material, and combinations thereof. Typically, the continuous-filament thread body is made of a polymer. The term "polymer" as used according to the invention may include copolymers, in particular random copolymers, alternating copolymers, graft copolymers and/or block copolymers. Further, as used according to the invention, the term "copolymer" relates to a polymer that is composed of at least two different monomer units. Accordingly, it may be within the scope of the present invention that the continuous-filament thread body is also made from terpolymers, tetrapolymers or the like.

Preferably, the continuous-filament thread body is made of a polyhydroxyalkanoate, a copolymer thereof or a mixture thereof. More specifically, the continuous-filament thread body is preferable made of a bio-absorbable material that is selected from the group consisting of polylactide, polyglycolide, polydioxanone, polytrimethylene carbonate, poly-ε-caprolactone, poly-3-hydroxybutyrate, poly-4-hydroxybutyrate, copolymers thereof and mixtures thereof.

In another embodiment, the continuous-filament thread body is made of a non-absorbable material that is selected from the group consisting of polyamide, polyester such as polyethylene terephthalate, polypropylene terephthalate and/or polybutylene terephthalate, polyethylene, polypropylene, polyurethane, polytetrafluoroethylene, in particular expanded polytetrafluorethylene, polyvinylidene difluoride, polytetrafluoropropylene, polyhexafluoropropylene, polyetherester, copolymers thereof, and mixtures thereof.

Furthermore, the continuous-filament thread body may comprise additives such as biological agents, medical agents, pharmaceutical agents, cells, and combinations thereof. Biological active agents may be selected from the group consisting of differentiation factors, growth factors, recruiting factors, adhesion factors, and combinations thereof. Appropriate growth factors may be selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insuline-like growth factor (IGF), hepatocyte growth factor (HGF), interleucin-1 B (IL-1 B), interleucin-8 (IL-8), nerve growth factor (NGF), and combinations thereof. Medical and pharmaceutical agents, respectively may be selected from the group consisting of antimicrobial agents, in particular antibiotic agents, disinfecting agents, growth-promoting agents, anti-inflammatory agents, analgetic agents, odor-controlling agents, and mixtures thereof. Preferred cellular additives may be somatic cells, in particular autologous cells, like fibroblasts, chondrocytes and/or precursors cells, in particular stem cells, for example. Furthermore, such substances, like collagen and/or hyaluronic acid for example, may be particularly beneficial in the field of plastic surgery to achieve cosmetically satisfactory results, like a smoothing of wrinkles, for example.

In a further embodiment, the continuous-filament thread body may be present in a drawn or undrawn state.

In a further embodiment, the continuous-filament thread body may be present in a sterile form.

The schematic figures show:
- Fig. 1: an embodiment of the continuous-filament thread not according to the invention,
- Fig. 2: a further embodiment of the continuous-filament thread not according to the invention,
- Fig. 3a: an embodiment of the barbed suture and the surgical needle and barbed suture combination, respectively not according to the invention,
- Fig. 3b: a further embodiment of the barbed suture and the surgical needle and barbed suture combination, respectively not according to the invention,
- Fig. 3c: a further embodiment of the barbed suture and surgical needle and barbed suture combination, respectively not according to the invention,
- Fig. 3d: a further embodiment of the barbed suture and surgical needle and barbed suture combination, respectively not according to the invention,
- Fig. 4: a further embodiment of the barbed suture and surgical needle and barbed suture combination, respectively not according to the invention,
- Fig. 5: further embodiment of the barbed suture and surgical needle and barbed suture combination, respectively not according to the invention,
- Fig. 6: a field of application for an embodiment of the surgical needle and barbed suture combination not according to the invention.

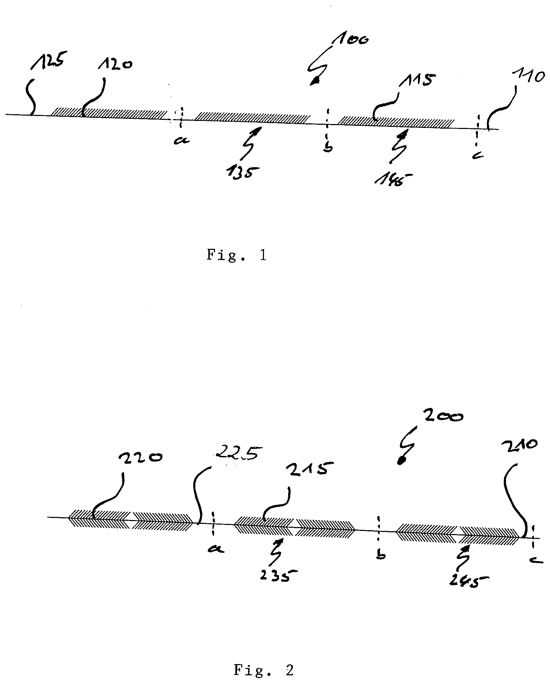

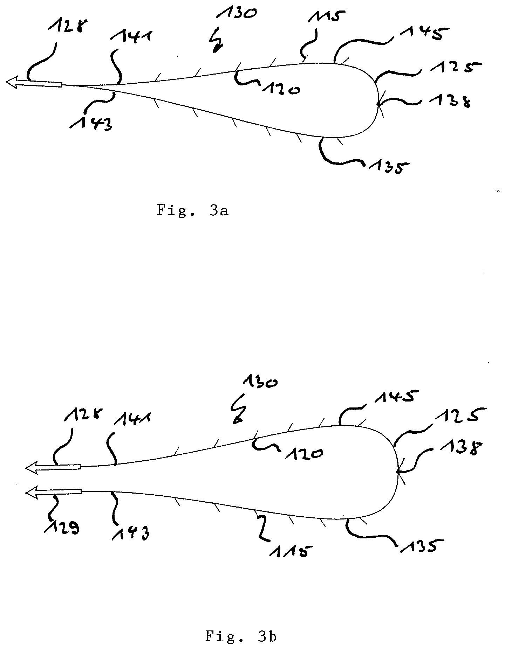

Fig. 1 discloses a continuous-filament thread 100 comprising a continuous-filament thread body 110. The continuous-filament thread body 110 comprises a plurality of barbs 115 projecting from the continuous-filament thread body 110. The barbs 115 are arranged in a unidirectional disposition on the continuous-filament thread body 110. More specifically, the continuous-filament thread body 110 comprises sections 120 having barbs - in the following referred to as barbed sections and sections 125 having no barbs - in the following referred to as barbless sections. The barbed sections 120 are interspaced by the barbless sections 125 and vice versa. The barbless sections 125 may advantageously serve as potential cutting areas for producing barbed sutures. For example, by cutting the continuous-filament thread body 110 at the dotted positions a-c, barbed sutures bodies 135, 145 may be produced as integral entities, i.e. in one piece, having unidirectionally arranged barbs. The suture bodies 135, 145 may be also joined in a subsequent step yielding a barbed suture 130 comprising bidirectionally arranged barbs as depicted in figure 3a.

Fig. 2 discloses a continuous-filament thread 200 comprising a continuous-filament thread body 210 comprising a plurality of barbs 215, which project from the continuous-filament thread body 210. The barbs 215 are arranged bidirectionally on the continuous-filament thread body 210. More specifically, the continuous-filament thread body 210 comprises barbed sections 220 having barbs and barbless sections 225, wherein the barbed sections 220 are interspaced by the barbless sections 225 and vice versa. By cutting the continuous-filament thread 210 at the dotted positions a-c, suture bodies 235, 245 having bidirectionally arranged barbs may be produced as integral entities, i.e. in one piece. The suture bodies 235, 245 may be joined in a subsequent step yielding a barbed suture comprising bidirectionally arranged barbs.

Fig. 3a shows a combination of a surgical needle 128 (depicted as an arrow) and a barbed suture 130 that comprises two suture bodies 135, 145 that are joined by a common junction 138 such as a knot. Both suture bodies 135, 145 comprise a plurality of unidirectionally arranged barbs 115, wherein the barbs 115 of the first suture body 135 are orientated in the direction of the second suture body 145 and the barbs 115 of the second suture body 145 are orientated in the direction of the first suture body 135. Thus, the barbed suture 130 in total comprises bidirectionally arranged barbs.

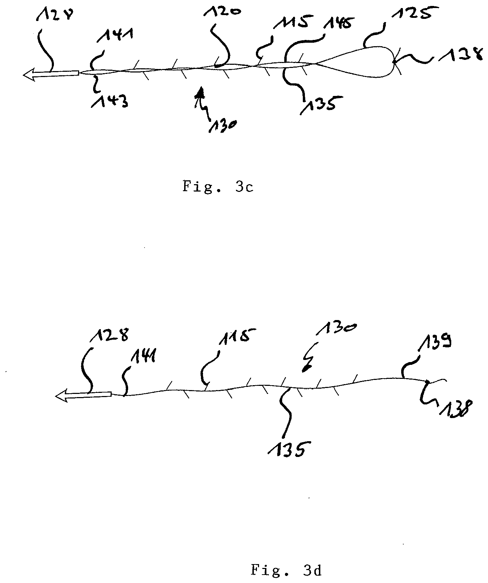

The barbed suture comprises barbed sections 120 and barbless sections 125. The barbless sections 125 are present around the common junction 138 and at the terminal sections 141, 143 of the suture 130. The terminal sections 141, 143 are attached to the surgical needle 128, preferably forming a suture loop as disclosed in figure 3.

The common junction 138 serves advantageously as a reference or control point for the surgeon in order to facilitate the identification of the suture's centre. Furthermore, the common junction 138 may serve as a control element in order to avoid an accidental sliding of the barbed suture 130 beyond its centre.

Fig. 3b discloses a further embodiment of a surgical needle and a barbed suture combination. The terminal sections 141, 143 of the barbed suture 130 are attached to the surgical needles 128, 129, preferably forming a loop. The surgical needles 128,129 may be of the same type or may stand for different needle types. With respect to further details and advantages, reference is made in its entirety to the description belonging to figure 3a.

Fig. 3c discloses a further embodiment of a surgical needle and a barbed suture combination. The barbed suture 130 comprises two suture bodies 135, 145 that are joined by a mutual junction 138. Both suture bodies 135, 145 comprise a section 120 of unidirectionally arranged barbs 115, wherein the barbed sections 120 are interspaced by a barbless section 125, wherein the barbless section 125 is around the mutual junction 138. Furthermore, the barbed suture 130 comprises terminal sections 141, 143 that are free of barbs and that are attached to the surgical needle 128. Besides, the suture bodies 135, 145 are twisted along the barbed sections 120. With respect to further details and advantages, reference is made in its entirety to the description belonging to figure 3a.

Fig. 3d discloses a further embodiment of a surgical needle and barbed suture combination. The barbed suture 130 comprises a suture body 135 having a plurality of unidirectionally arranged barbs 115. The barbs 115 are present one the suture body 135 in form of two unidirectional dispositions. In relation to one barb disposition, the other disposition is formed on the suture body 135 at approximately 180 ° in the circumferential direction and offset in relation to the first unidirectional disposition. The barbed suture 130 comprises a junction 138 such as a knot at one terminal section 139 of the suture body 135. The opposite terminal section 141 is attached to a surgical needle 128. With respect to further details and advantages, reference is made in its entirety to the description belonging to figure 3a.

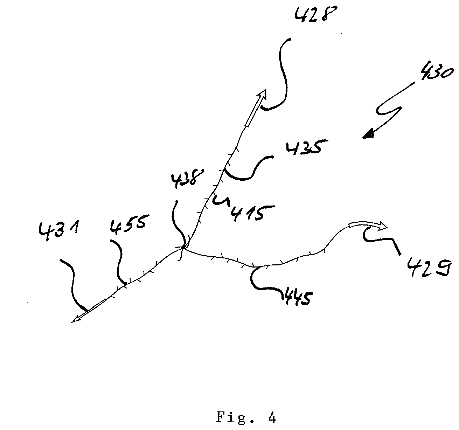

Fig. 4 shows a further embodiment of a surgical needle and barbed suture combination. The barbed suture 430 comprises three suture bodies 435, 445, 455, wherein the suture bodies 435, 445, 455 comprise a plurality of barbs 415 and wherein the suture bodies 435, 445, 455 are joined by a mutual junction 438. The suture bodies 435, 445, 455 are roughly directed in the edges of a triangle. The mutual junction 438 may be a knot or as an alternative a T-fitting. The suture bodies 435, 445, 455 are attached to the surgical needles 428, 429, 431 as disclosed in Fig. 4. With respect to the surgical needles 428, 429, 431, needles of the same or a different type may be used. Furthermore, the suture bodies 435, 445, 455 may comprise barbed and/or unbarbed sections. It is further within the scope of the present invention that some of the suture bodies are free of barbs 415. With respect to further features and advantages, reference is made in its entirety to the previous figure descriptions.

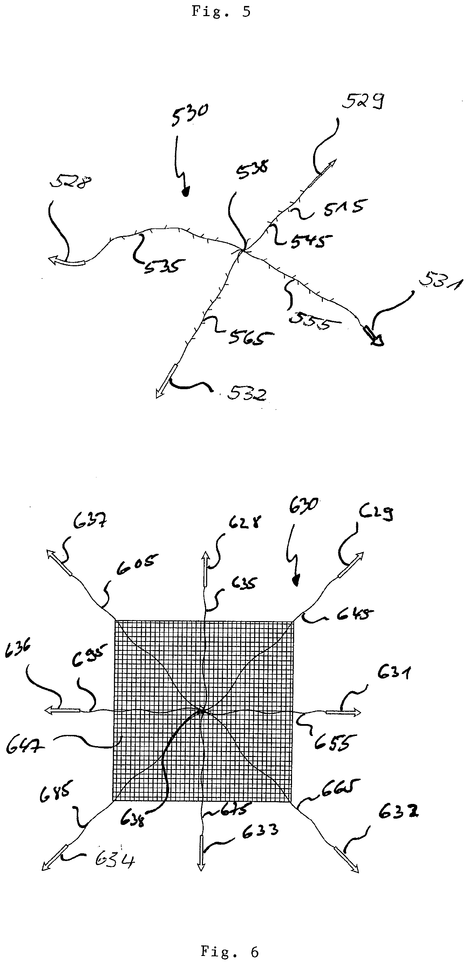

Fig. 5 shows a further embodiment of a surgical needle and barbed suture combination. The barbed suture 530 comprises four suture bodies 535, 545, 555, 565, wherein the suture bodies 535, 545, 555, 565 comprise a plurality of barbs 515 and wherein the suture bodies 535, 545, 555 are joined by a mutual junction 538. The suture bodies 535, 545, 555, 565 are roughly directed in the edges of a square. The mutual junction 538 may be a knot. The suture bodies are attached to the surgical needles 528, 529, 531, 532. With respect to further features and advantages reference is made in its entirety to the previous figure descriptions.

Fig. 6 shows a star-like arranged barbed suture 630, wherein the barbs for sake of clearness are not depicted. The barbed suture comprises suture bodies 605, 635, 645, 655, 665, 675, 685, 695 being attached to the surgical needles 628, 629, 631, 632, 633, 634, 636, 637. The barbed suture 630 may be, by way of example, used to fix a medical mesh 647 in the body of the patient.

Instead of the junction described in the above figure descriptions, a resistance element may also be used.

### Example (not according to the invention)

A polydioxanone monofilament is extruded, passed through a quench bath and wound on a drum winder without drawing the fiber to its final length. Subsequently the undrawn fiber is passed through a cutting device to produce an undrawn, continuous monofilament having a regular pattern of unidirectional barbed sections followed by sections that have no barbs. Afterwards this continuous barbed monofilament is drawn to its final length by passing it through multistage drawing equipment, applying a 3-fold drawing ratio and then wound on a spool. In a next step, the fiber is wound off the spool and cut in the middle of each unbarbed (barbless) section, resulting in an amount of monofilament strands having an unidirectional barbed section in the center and non-barbed sections at the ends of the strands.

These strands are then used to produce a star shaped fixation device for surgical meshes. Therefore four of these unidirectional strands as mentioned above are connected on one end by a knot, in a way that the directions of the barbs of the respective fibres points towards the knot. In a second step the other free fibre ends are attached to a needle resulting in the desired device (see Figure 5).

## Claims

1. Use of a continuous-filament thread (100; 200) for the production of different barbed sutures, wherein the continuous-filament thread (100; 200) comprises a continuous-filament thread body (110; 210) comprising a plurality of barbs (115; 215) projecting from the thread body (110; 210), wherein the continuous-filament thread body (110; 210) is unprocessed at least in view of its length, wherein the barbs (115; 215) have an alternating or random configuration in view of barb design, and the barbs (115; 215) are arranged in an alternating or random disposition on the continuous-filament thread body (110; 210), wherein the disposition is selected from the group consisting of a row disposition, a staggered disposition, an overlapping disposition, an offset disposition, an offset and partially overlapping disposition, a zigzag disposition, a random or arbitrary disposition, a meander-like disposition, a serpentine-like disposition, a sinus-like disposition, a spiral disposition, a helical disposition, and combinations thereof, and wherein the continuous-filament thread body (110; 210) is a hollow continuous-filament thread body.

2. Use of a continuous-filament thread (100; 200) for the production of different barbed sutures according to claim 1, **characterized in that** the barbs (115; 215) have a shape that is selected from the group consisting of escutcheon-shape, shield-shape, scale-shape, wedge-shape, thorn-shape, W-shape, arrow-shape, spike-shape, tin-shape V-shape and, combinations thereof.

3. Use of a continuous-filament thread (100; 200) for the production of different barbed sutures according to claim 1 or 2, **characterized in that** the barbs (115; 215) are arranged in a unidirectional disposition or in a multidirectional, preferably bidirectional, disposition on the continuous-filament thread body (110; 210), wherein the continuous-filament thread body preferably comprises repeating units of unidirectional or multidirectional, preferably bidirectional, arranged barbs (115; 215).

4. Use of a continuous-filament thread (100; 200) for the production of different barbed sutures according to one of the preceding claims, **characterized in that** the continuous-filament thread body (110; 210) comprises sections (125; 225) that are free of barbs.

5. Use of a continuous-filament thread (100; 200) for the production of different barbed sutures according to one of the preceding claims, **characterized in that** the continuous-filament thread body (110; 210) comprises sections (120; 220) having barbs (115; 215) that are interspaced by sections (125; 225) having no barbs.

6. Use of a continuous-filament thread (100; 200) for the production of different barbed sutures according to one of the preceding claims, **characterized in that** the continuous-filament thread body (110; 210) is a tubular continuous-filament thread body.

## Patentansprüche

1. Verwendung einer Endlosfaser (100; 200) zur Herstellung von unterschiedlich mit Widerhaken versehenem Nahtmaterial, wobei die Endlosfaser (100; 200) einen Endlosfaserkörper (110; 210) umfasst, der eine Mehrzahl von vom Endlosfaserkörper (110; 210) abstehenden Widerhaken (115; 215) umfasst, wobei der Endlosfaserkörper (110; 210) zumindest in Hinblick auf seine Länge unbearbeitet ist, wobei die Widerhaken (115; 215) eine alternierende oder beliebige Konfiguration in Hinblick auf die Widerhakengestaltung aufweisen, und die Widerhaken (115; 215) in einer alternierenden oder beliebigen Anordnung am Endlosfaserkörper (110; 210) angeordnet sind, wobei die Anordnung ausgewählt ist aus der Gruppe bestehend aus reihenförmige Anordnung, gestaffelte Anordnung, überlappende Anordnung, versetzte Anordnung, versetzte und teilweise überlappende Anordnung, Zickzack-Anordnung, beliebige oder willkürliche Anordnung, mäanderartige Anordnung, serpentinenartige Anordnung, sinusartige Anordnung, spiralförmige Anordnung, wendelförmige Anordnung und Kombinationen davon, und wobei der Endlosfaserkörper (110; 210) als hohler Endlosfaserkörper ausgebildet ist.

2. Verwendung einer Endlosfaser (100; 200) zur Herstellung von unterschiedlich mit Widerhaken versehenem Nahtmaterial nach Anspruch 1, **dadurch gekennzeichnet, dass** die Widerhaken (115; 215) eine Form aufweisen, die ausgewählt ist aus der Gruppe bestehend aus wappenförmig, schildförmig, schuppenförmig, keilförmig, dornförmig, W-förmig, pfeilförmig, stachelförmig, zinnenförmig, V-förmig und Kombinationen davon.

3. Verwendung einer Endlosfaser (100; 200) zur Herstellung von unterschiedlich mit Widerhaken versehenem Nahtmaterial nach Anspruch 1 oder 2, **dadurch gekennzeichnet, dass** die Widerhaken (115; 215) in einer unidirektionalen Anordnung oder in einer multidirektionalen, bevorzugt bidirektionalen, Anordnung auf dem Endlosfaserkörper (110; 210) angeordnet sind, wobei der Endlosfaserkörper bevorzugt sich wiederholende Einheiten von unidirektional oder multidirektional, bevorzugt bidirektional, angeordneten Widerhaken (115; 215) umfasst.

4. Verwendung einer Endlosfaser (100; 200) zur Herstellung von unterschiedlich mit Widerhaken versehenem Nahtmaterial nach einem der vorhergehenden Ansprüche, **dadurch gekennzeichnet, dass** der Endlosfaserkörper (110; 210) Abschnitte (125; 225) umfasst, die von Widerhaken frei sind.

5. Verwendung einer Endlosfaser (100; 200) zur Herstellung von unterschiedlich mit Widerhaken versehenem Nahtmaterial nach einem der vorhergehenden Ansprüche, **dadurch gekennzeichnet, dass** der Endlosfaserkörper (110; 210) Abschnitte (120; 220) mit Widerhaken (115; 215) umfasst, die dazwischenliegende Abschnitte (125; 225) ohne Widerhaken aufweisen.

6. Verwendung einer Endlosfaser (100; 200) zur Herstellung von unterschiedlich mit Widerhaken versehenem Nahtmaterial nach einem der vorhergehenden Ansprüche, **dadurch gekennzeichnet, dass** der Endlosfaserkörper (110; 210) als schlauchförmiger Endlosfaserkörper ausgebildet ist.

## Revendications

1. Utilisation d'un fil à filaments continus (100 ; 200) pour la production de différentes sutures à barbes, dans laquelle le fil à filaments continus (100 ; 200) comprend un corps de fil à filaments continus (110 ; 210) comprenant une pluralité de barbes (115 ; 215) faisant saillie depuis le corps de fil (110 ; 210), dans laquelle le corps de fil à filaments continus (110 ; 210) est non traité au moins en ce qui concerne sa longueur, dans laquelle les barbes (115 ; 215) ont une configuration alternée ou aléatoire en terme de conception de barbes, et les barbes (115 ; 215) sont agencées dans une disposition alternée ou aléatoire sur le corps de fil à filaments continus (110 ; 210), dans laquelle la disposition est choisie dans le groupe constitué par une disposition en rangées, une disposition en quinconce, une disposition superposée, une disposition décalée, une disposition décalée et partiellement superposée, une disposition en zigzags, une disposition aléatoire ou arbitraire, une disposition en forme de méandres, une disposition en forme de serpentin, une disposition de type sinusoïdal, une disposition en spirale, une disposition hélicoïdale, et les combinaisons de celles-ci, et dans laquelle le corps de fil à filaments continus (110 ; 210) est un corps de fil à filaments continus creux.

2. Utilisation d'un fil à filaments continus (100 ; 200) pour la production de différentes sutures à barbes selon la revendication 1, **caractérisée en ce que** les barbes (115 ; 215) ont une forme qui est choisie dans le groupe constitué par une forme d'écusson, une forme de bouclier, une forme d'écaille, une forme de coin, une forme d'épine, une forme en W, une forme de flèche, une forme de pointe, une forme de boîte, une forme en V, et les combinaisons de celles-ci.

3. Utilisation d'un fil à filaments continus (100 ; 200) pour la production de différentes sutures à barbes selon la revendication 1 ou 2, **caractérisée en ce que** les barbes (115 ; 215) sont agencées dans une disposition unidirectionnelle ou dans une disposition multidirectionnelle, de préférence bidirectionnelle sur le corps de fil à filaments continus (110 ; 210), dans laquelle le corps de fil à filaments continus comprend de préférence des motifs répétitifs de barbes à agencement unidirectionnel ou multidirectionnel, de préférence bidirectionnel (115 ; 215).

4. Utilisation d'un fil à filaments continus (100 ; 200) pour la production de différentes sutures à barbes selon une des revendications précédentes, **caractérisée en ce que** le corps de fil à filaments continus (110 ; 210) comprend des sections (125 ; 225) qui sont dépourvues de barbes.

5. Utilisation d'un fil à filaments continus (100 ; 200) pour la production de différentes sutures à barbes selon une des revendications précédentes, **caractérisée en ce que** le corps de fil à filaments continus (110 ; 210) comprend des sections (120 ; 220) ayant des barbes (115 ; 215) qui sont entrecoupées de sections (125 ; 225) n'ayant pas de barbes.

6. Utilisation d'un fil à filaments continus (100 ; 200) pour la production de différentes sutures à barbes selon une des revendications précédentes, **caractérisée en ce que** le corps de fil à filaments continus (110 ; 210) est un corps de fil à filaments continus tubulaire.
